# EUROPEAN PATENT SPECIFICATION

(11) **EP 3 402 537 B1**
(45) Date of publication and mention of the grant of the patent: **06.01.2021**
(21) Application number: 17738231.4
(22) Date of filing: 13.01.2017
(51) Int. Cl.: A61K 101/02, A61K 38/48, A61K 51/08, C12N 9/64

(54) **QUANTITATIVE PET IMAGING OF TISSUE FACTOR EXPRESSION USING 18F-LABELLED ACTIVE SITE INHIBITED FACTOR VII**
QUANTITATIVE PET-BILDGEBUNG DER GEWEBEFAKTOREXPRESSION MITTELS 18F-MARKIERTEM, DURCH AN DER AKTIVEN STELLE GEHEMMTEN FAKTOR VII
IMAGERIE PAR TEP QUANTITATIVE DE L'EXPRESSION DU FACTEUR TISSULAIRE EMPLOYANT UN FACTEUR VII À SITE ACTIF INHIBÉ MARQUÉ AU 18F

(30) Priority: 15.01.2016 DK 201670019
(43) Date of publication of application: 21.11.2018
(73) Proprietor: Rigshospitalet, 2100 Copenhagen (DK)
(72) Inventor: KJAER, Andreas, 2000 Frederiksberg (DK); HAAGEN NIELSEN, Carsten, 2200 Copenhagen N (DK)
(74) Representative: Budde Schou A/S
(86) International application number: PCT/DK2017/050006
(87) International publication number: WO 2017/121436

(56) References cited:
- WO-A1-01/83725
- WO-A2-02/22776
- WO-A2-2004/007557
- WO-A2-2004/064870
- Tom Knudsen ET AL: "Tisssue factor and factor viia cross-species compatibility Tisssue factor and factor viia cross-species compatibility", , 1 June 2011 (2011-06-01), XP055606616, Retrieved from the Internet: URL:https://www.bioscience.org/fbs/getfile .php?FileName=/2011/v16/af/3906/3906.pdf?b frame=PDFII [retrieved on 2019-07-17]
- NIELSEN, C.H. ET AL.: 'Quantitative PET Imaging of Tissue Factor Expression Using 18F-Labeled Active Site-Inhibited Factor VII' THE JOURNAL OF NUCLEAR MEDICINE vol. 57, no. 1, 2016, pages 89 - 95, XP055401414
- ERLANDSSON, M. ET AL.: 'Synthesis and characterization of 18F-labeled active site inhibited factor VII (ASIS' JOURNAL OF LABELLED COMPOUNDS AND RADIOPHARMACEUTICALS vol. 58, no. 5, 2015, pages 196 - 201, XP055401416

## Description

### FIELD OF THE INVENTION

The present invention relates to a positron-emitting 18-F labelled Factor VII for noninvasive PET imaging of Tissue Factor expressing tumors in humans, as defined in the claims. More specifically the invention relates to human TF PET imaging of any solid cancer disease for diagnosis, staging, treatment monitoring, companion diagnostics and especially as an imaging biomarker for predicting prognosis, progression and recurrence.

### BACKGROUND OF THE INVENTION

Tissue factor (TF) is a 47 kDa transmembrane protein, which binds factor VII (FVII) with high affinity. The resulting complex initiates the extrinsic coagulation cascade essential for normal hemostasis. Upon binding to TF, the zymogen FVII gets activated to the serine protease, FVIIa; and the TF:FVIIa complex further activates factor X eventually leading to thrombin generation and hemostasis.

In addition to its role in coagulation, TF plays a central role in cancer progression, angiogenesis, invasion and hematogeneous metastatic dissemination. Many tumors express various levels of cells surface TF, and the TF:FVIIa complex has been shown to activate protease activated receptor 2 (PAR2), and through intracellular signaling to induce an anti-apoptotic effect as well as to enhance tumor growth, migration and angiogenesis. In addition, TF:FVIIa more indirectly facilitates metastatic dissemination through thrombin generation and PAR1 signaling (*1-4*).

Clinically, TF is overexpressed in a number of cancers including glioma, breast, colorectal, prostate, and pancreatic cancer (*5-8*). Within breast, gastric, esophageal, liver, colorectal and pancreatic cancer it has been shown that TF, measured by immunohistochemistry, is associated with increased metastatic disease and is a prognostic marker of poor overall survival (*1*).

Targeting TF has proven effective as a cancer therapy in preclinical models. Yu et al. demonstrated that silencing of TF by siRNA reduced tumor growth in a mouse model of colorectal cancer (*11*). Using an immunoconjugate with FVII as the binding domain, Hu et al. suppressed tumor growth in a human melanoma xenograft mouse model (*12*). Ngo et al. and Versteeg et al. demonstrated that anti-TF antibodies inhibited metastasis in an experimental metastasis model and suppressed tumor growth in a breast cancer model *(13,14).*

Targeting of TF with an antibody-drug conjugate (ADC) was recently shown to have a potent and encouraging therapeutic effect in murine cancer models, including patient-derived xenografts models (*15*). A non-invasive method for specific assessment of tumor TF expression status would be valuable. Such a tool would be clinically relevant for guidance of patient management and as companion diagnostics for emerging TF-targeting therapies.

Normally, TF is constitutively expressed on the surface of many extravascular cell types that are not in contact with the blood, such as fibroblasts, pericytes, smooth muscle cells and epithelial cells, but not on the surface of cells that come in contact with blood, such as endothelial cells and monocytes. However, TF is also expressed in various pathophysiological conditions where it is believed to be involved in progression of disease states within cancer, inflammation, atherosclerosis and ischemia/reperfusion. Thus, TF is now recognised as a target for therapeutic intervention in conditions associated with increased expression.

FVIIa is a two-chain, 50 kilodalton (kDa) vitamin-K dependent, plasma serine protease which participates in the complex regulation of in vivo haemostasis. FVIIa is generated from proteolysis of a single peptide bond from its single chain zymogen, Factor VII (FVII), which is present at approximately 0.5 µg/ml in plasma. The zymogen is catalytically inactive. The conversion of zymogen FVII into the activated two-chain molecule occurs by cleavage of an internal peptide bond. In the presence of calcium ions, FVIIa binds with high affinity to exposed TF, which acts as a cofactor for FVIIa, enhancing the proteolytic activation of its substrates FVII, Factor IX and FX.

In addition to its established role as an initiator of the coagulation process, TF was recently shown to function as a mediator of intracellular activities either by interactions of the cytoplasmic domain of TF with the cytoskeleton or by supporting the FVIIa-protease dependent signaling. Such activities may be responsible, at least partly, for the implicated role of TF in tumor development, metastasis and angiogenesis. Cellular exposure of TF activity is advantageous in a crisis of vascular damage but may be fatal when exposure is sustained as it is in these various diseased states. Thus, it is critical to regulate the expression of TF function in maintaining the health.

Radiolabelled TF agonists and/or TF antagonists may be valuable for diagnostic imaging with a gamma camera, a PET camera or a PET/CT camera, in particular for the evaluation of TF expression of tumor cells, for grading the malignancy of tumor cells known to express TF receptors, for the monitoring of tumors with TF expression during conventional chemotherapy or radiation therapy. Also TF agonists and/or TF antagonists labelled with alpha- or beta-emitting isotopes could be used for therapy, possibly with bi-specific binding to compounds with chemotherapeutic action, which may be related to the presence of TF receptors. In those cases the diagnostic imaging may be important for the evaluation of tumor response expected after therapy with TF receptor binding drugs.

Also other kinds of diseases with increased expression of surface accessible TF receptors may be observed, maybe inflammatory or auto-immune diseases, where both diagnostic and therapeutic application of radiolabelled TF agonists and/or TF antagonists may become relevant.

One example of a TF antagonist, inactivated FVII (FVIIai) is FVIIa modified in such a way that it is catalytically inactive. Thus, FVllai is not able to catalyze the conversion of FX to FXa, or FIX to FIXa but still able to bind tightly to TF in competition with active endogenous FVIIa and thereby inhibit the TF function.

International patent applications WO 92/15686, WO 94/27631, WO 96/12800, WO 97/47651 relates to FVllai and the uses thereof. International patent applications WO 90/03390, WO 95/00541, WO 96/18653, and European Patent EP 500800 describes peptides derived from FVIIa having TF/FVIIa antagonist activity. International patent application WO 01/21661 relates to bivalent inhibitor of FVII and FXa.

Hu Z and Garen A (2001) Proc. Natl. Acad. Sci. USA 98; 12180-12185, Hu Z and Garen A (2000) Proc. Natl. Acad. Sci. USA 97; 9221-9225, Hu Z and Garen A (1999) Proc. Natl. Acad. Sci. USA 96; 8161-8166, and International patent application WO 0102439 relates to immunoconjugates which comprises the Fc region of a human IgG1 immunoglobulin and a mutant FVII polypeptide, that binds to TF but do not initiate blood clotting.

Furthermore, International patent application WO 98/03632 describes bivalent agonists having affinity for one or more G-coupled receptors, and Burgess, L.E. et al., Proc. Natl. Acad. Sci. USA 96, 8348-8352 (July 1999) describes "Potent selective non-peptidic inhibitors of human lung tryptase".

WO 2004/064870 discloses various conjugates of TF agonists and TF antagonists linked to a radionuclide for use in diagnostics. WO 2004/007557 discloses TF antagonists linked to a cytotoxic domain, which binds to and inhibits the activity of TF and induces a cytotoxic response, as well as their use in the prophylaxis or treatment of diseases related to TF functions. WO 01/83725 and WO 02/22776 discloses human Factor VIIa polypeptide variants with coagulant activity comprising the amino acid sequence of native human coagulation Factor VII wherein of one or more specific amino acids has been replaced.

Knudsen T. et al. (2011, retrieved from the internet URL:https://www bioscience.org/fbs/getfile.php?FileName=/2011/v16/af/3906/3906.pdf?bframe=PDFII) provides a review on the features of human TF and FVIIa, their intermolecular interactions, and the biological effects of TF-FVIIa complex formation and compares this to findings obtained in studies addressing TF or FVIIs of non-human origin. It is for instance disclosed that whereas dog studies closely recapitulate the human setting, the incompatibility of human FVIIa with mouse TF is an obstacle to address questions about TF-FVIIa interactions in murine models.

Erlandsson M. et al. (THE JOURNAL OF NUCLEAR MEDICINE, 2015, Vol 58, no. 5, pages 196-201) discloses the synthesis of 18F-labeled active site inhibited factor VII and tests its potential use as a positron emission tomography tracer to image TF expression.

NIELSEN C.H. et al.(THE JOURNAL OF NUCLEAR MEDICINE, 2016, Vol 57, no. 1, pages 89-95) discloses the use of the imaging agent 18F-labeled human active siteinhibited factor VII in Positron Emission Tomography (PET) for identification and quantification of tissue factor expressing cancers in mice. The authors conclude in this paper that while 18F-FVIIai is a promising PET tracer for specific and noninvasive imaging of tumor TF expression the tracer merits further development and clinical translation, with potential to become a companion diagnostics for emerging TF targeted therapies.

There is still a need in the art for improved compounds, which efficiently inhibits pathophysiological TF function at relatively low doses and which does not produce undesirable side effects.

### SUMMARY OF THE INVENTION

The invention is defined by the claims. Any subject-matter falling outside the scope of the claims is provided for information purposes only. Any references in the description to methods of treatment, diagnosis or surgery refer to the compounds, pharmaceutical compositions and medicaments of the present invention for use in a method for treatment, diagnosis or surgery of the human or animal body by therapy. The present inventors have surprisingly found that 18-F labelled factor Vllai (Egtl 18F-ASIS) is very useful for PET imaging of a Tissue Factor expressing tumor in a human. So far 18-F labelled human factor VIIa has only been validated in mice implanted with human xenografts (cf above mentioned paper). Although the human factor VIIa binds to such xenografts the mouse model is not a representative model for human use, since human factor VIIa does hardly bind to the murine TF. Accordingly, background signals from normal murine tissue is very low in such an animal model rendering any conclusion as to the applicability in humans highly speculative. In order to address the true applicability of 18-F labelled factor VIIa for PET scanning of tumors the present inventors have demonstrated in a murine and dog model (which is representative for human use since the used ligand binds strongly to canine TF) that the background signal surprisingly is sufficiently low for obtaining PET images useful for diagnostic purposes.

Specifically, the present invention provides a positron-emitting 18-F labelled factor VIIa (FVIIa) imaging agent for use in a diagnosis by PET imaging of a Tissue Factor expressing tumor in a human, said agent comprising native human FVIIa or a variant thereof radiolabeled with 18-F, wherein the agent is to be administered in a dose of 50-400 MBq followed by PET scanning 1-6 hours after the agent has been administered, quantification through SUVmax and/or SUVmean for thereby obtaining a PET image for the diagnosis of tumor tissue factor status for use of diagnosis, treatment monitoring or as a companion diagnostics based on target-to-background ratio or absolute uptake (SUV).

Preferably FVIIa is active site inhibited factor VIIa (FVIIai) of SEQ ID NO: 1. In a particularly preferred embodiment FVllai has been labelled with N-succinimidyl 4-18Ffluorobenzoate (18F-SFB) to produce:

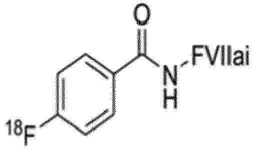

As elaborated on in the experimental part of the present disclosure the imaging agent of the invention is particularly useful in diagnosing breast, gastric, esophageal, liver, colorectal and pancreatic cancer.

In a further aspect the present invention provides a method for generating images of tissue factor expression in a human by diagnostic imaging involving administering the imaging agent of the invention to the human, and generating an image of at least a part of said body to which said imaging agent is administered.

The present invention is therefore also directed to a method of diagnosing by PET imaging a Tissue Factor expressing tumor in a human, said method comprising:
- administering a positron-emitting 18-F labelled factor VIIa (FVIIa) imaging agent, said agent comprising native human FVIIa or a variant thereof radiolabeled with 18-F, wherein the agent is administered in a dose of 50-400 MBq followed by PET scanning 1-6 hours after the agent has been administered, quantification through SUVmax and/or SUVmean for obtaining a PET image for the diagnosis of tumor tissue factor status.

Preferably, the method further includes the step of treatment monitoring or as a companion diagnostics based on target-to-background ratio or absolute uptake (SUV).

### BRIEF DESCRIPTION OF THE DRAWINGS

Figure 1 shows PET images of mice with subcutaneous human pancreatic xenograft tumors detected with murine ¹⁸F-FVIIai (A) and human 18F-FVIIai (B).
Figure 2 shows *ex vivo* bio-distribution of mice with BxPC-3 tumors performed 4 hours after injection of 18F-FVIIai of human or murine origin.

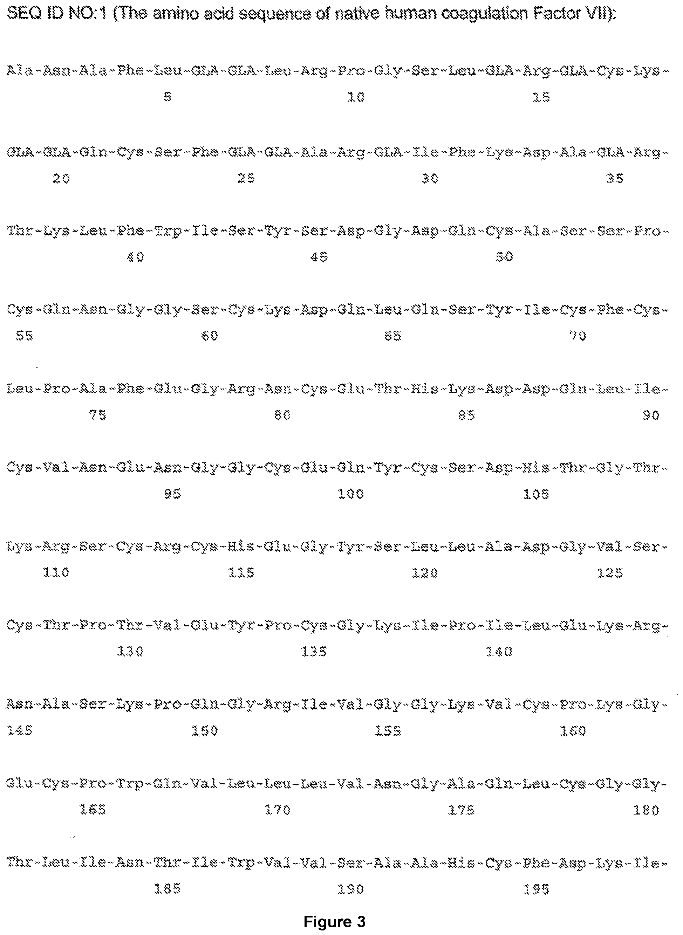

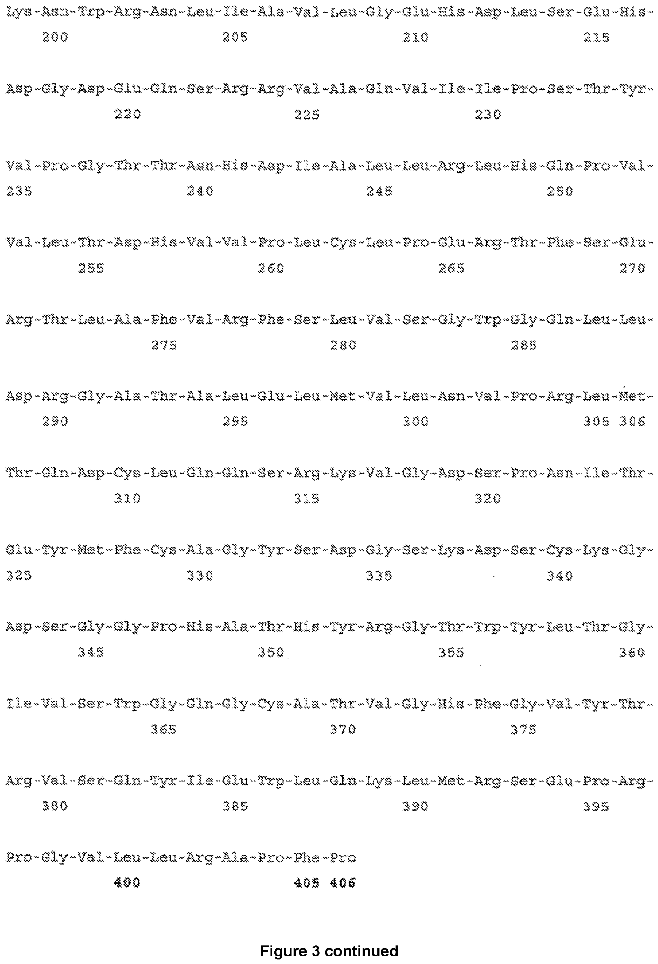

Figure 3 shows human Factor VII having the sequence of SEQ ID NO: 1.

### DETAILED DESCRIPTION OF THE INVENTION

The 18-F labelled factor VIIa imaging agents of the present invention may be synthesized and purified in accordance with international patent application WO2004064870.

The terms "variant" or "variants", as used herein, is intended to designate human Factor VII having the sequence of SEQ ID NO: 1, wherein one or more amino acids of the parent protein have been substituted by another amino acid and/or wherein one or more amino acids of the parent protein have been deleted and/or wherein one or more amino acids have been inserted in protein and/or wherein one or more amino acids have been added to the parent protein. Such addition can take place either at the N-terminal end or at the C-terminal end of the parent protein or both. In one embodiment of the invention the variant has a total amount of amino acid substitutions and/or additions and/or deletions independently selected from the group consisting of 1, 2, 3, 4, 5, 6, 7, 8, 9, and 10. The activation of factor VII to factor VIIa, involves the hydrolysis of a single peptide bond between Arg152 and Ile153, resulting in a two-chain molecule consisting of a light chain of 152 amino acid residues and a heavy chain of 254 amino acid residues held together by a single disulfide bond.

Preferably, the FVIIa of SEQ ID NO: 1 is active site inhibited factor VIIa (FVIIai) and modified in such a way that it is catalytically inactive, such as having the amino acid modification comprised of Ser344, Asp242, and His193.

By "catalytically inactivated in the active site of the FVIIa polypeptide" is meant that a FVIIa inhibitor is bound to the FVIIa polypeptide and decreases or prevents the FVIIa-catalysed conversion of FX to FXa. A FVIIa inhibitor may be identified as a substance, which reduces the amidolytic activity by at least 50% at a concentration of the substance at 400 µM in the FVIIa amidolytic assay described by Persson et al. (Persson et al., J. Biol. Chem. 272: 19919-19924 (1997)). Preferred are substances reducing the amidolytic activity by at least 50% at a concentration of the substance at 300 µM; more preferred are substances reducing the amidolytic activity by at least 50% at a concentration of the substance at 200 µM.

The "FVIIa inhibitor" may be selected from any one of several groups of FVIIa directed inhibitors. Such inhibitors are broadly categorised for the purpose of the present invention into i) inhibitors which reversibly bind to FVIIa and are cleavable by FVIIa, ii) inhibitors which reversibly bind to FVIIa but cannot be cleaved, and iii) inhibitors which irreversibly bind to FVIIa. For a review of inhibitors of serine proteases see Proteinase Inhibitors (Research Monographs in cell and Tissue Physiology; v. 12) Elsevier Science Publishing Co., Inc., New York (1990).

The FVIIa inhibitor moiety may also be an irreversible FVIIa serine protease inhibitor. Such irreversible active site inhibitors generally form covalent bonds with the protease active site. Such irreversible inhibitors include general serine protease inhibitors such as peptide chloromethylketones (see, Williams et al., J. Biol. Chem. 264:7536-7540 (1989)) or peptidyl cloromethanes; azapeptides; acylating agents such as various guanidinobenzoate derivatives and the 3-alkoxy-4-chloroisocoumarins; sulphonyl fluorides such as phenylmethylsulphonylfluoride (PMSF); diisopropylfluorophosphate (DFP); tosylpropylchloromethyl ketone (TPCK); tosyllysylchloromethyl ketone (TLCK); nitrophenyl-sulphonates and related compounds; heterocyclic protease inhibitors such as isocoumarines, and coumarins.

### EXAMPLE 1

The in vivo properties of 18F-FVIIai for PET imaging was evaluated in a mouse model of human pancreatic cancer using small animal PET/CT. The uptake of 18F-FVIIai measured by PET was correlated with TF expression measured *ex vivo* to confirm specific imaging of tumor TF expression.

Active site inhibited factor VIIa (FVIIai) was obtained by inactivation with phenylalanine-phenylalanine-arginine-chloromethyl ketone. FVIIai was radiolabeled with N-succinimidyl 4-18Ffluorobenzoate (18F-SFB) and purified. The corresponding product, 18F-FVIIai, was injected into nude mice with subcutaneous human pancreatic xenograft tumors (BxPC-3) and investigated using small animal PET/CT imaging 1, 2 and 4 hours after injection. Ex vivo biodistribution was performed after the last imaging session, and tumor tissue was preserved for molecular analysis. A blocking experiment was performed in a second set of mice. The expression pattern of TF in the tumors was visualized by immunohistochemistry and the amount of TF in tumor homogenates was measured by ELISA and correlated with the uptake of 18F-FVIIai in the tumors measured in vivo by PET imaging.

The PET images showed high uptake of 18F-FVIIai in the tumor regions with a mean uptake of 2.5 ± 0.3 percentage injected dose per gram (%ID/g) (Mean ± SEM) 4 hours after injection of 7.3-9.3 MBq 18F-FVIIai and with an average maximum uptake in the tumors of 7.1 ± 0.7 %ID/g at 4 hours. In comparison, the muscle uptake was 0.2 ± 0.01 %ID/g at 4 hours. At 4 hours the tumors had the highest uptake of any organ. Blocking with FVIIai significantly reduced the uptake of 18F-FVIIai from 2.9 ± 0.1 to 1.4 ± 0.1 %ID/g (P<0.001). The uptake of [18F]FVIIai measured *in vivo* by PET imaging correlated (r=0.72, P<0.02) with TF protein level measured *ex vivo.*

A limitation of the study is the absence of human TF outside the tumor regions in the xenograft mouse model applied. It has previously been shown that the cross species compatibility for TF/FVII is rather low such that human FVIIai binds with much lower affinity to murine than to human TF (28,33). Hence, the mouse model underestimates the background uptake of FVllai to be seen in human tissues.

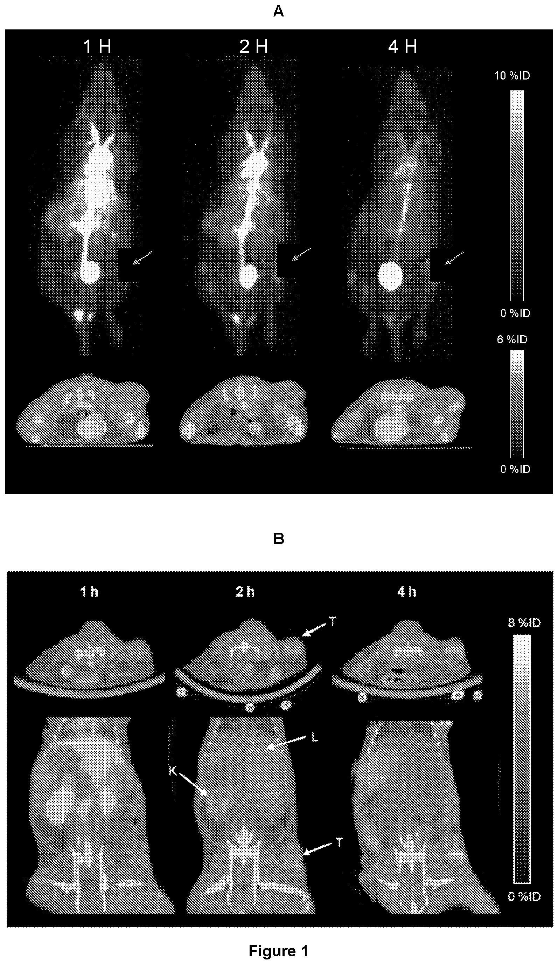

Referring to Figure 1A there is shown mice with tissue factor positive human xenograft tumors (BxPC-3) were injected with murine ¹⁸F-FVIIai and scanned with a dedicated small animal PET/CT scanner at 1, 2 and 4 hours after injection. 18F-FVIIai accumulates in the tissue factor positive tumor (arrows) despite the endogenous factor VII. Also, and surprisingly the background expression of tissue factor in the animal does not impede the visualization of the tissue factor positive tumor. In Figure 1B there is shown a corresponding experiment with 18F-FVIIai of human origin. The tumors are clearly visible (arrow) . The muscle uptake is still low and a good tumor to muscle contrast is obtained at 4 hours.

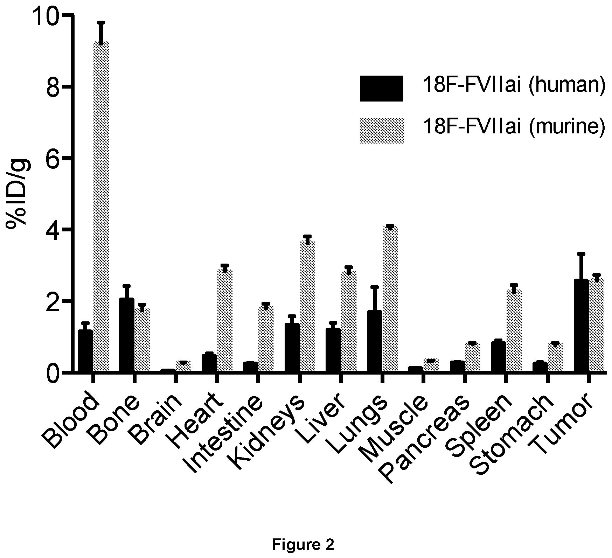

Referring to Figure 2 there is shown *ex vivo* biodistribution of mice with BxPC-3 tumors performed 4 hours after injection of 18F-FVIIai of human or murine origin. The uptake of murine 18F-FVIiai in the tumors is not affected by the ability to bind murine tissue factor expressed in normal tissue or by competition with endogenous factor VII. The results from the *ex vivo* biodistribution clearly demonstrate that the biodistribution using human 18F-FVIIai that does not bind to mouse tissue factor can in no way predict the bio-distribution in the presence of background binding outside the tumor (murine tissue factor).

Surprisingly, the radiolabeled protein of the present invention is very useful in the visualization of TF positive tumors despite the presence of TF background expression and competition with endogenous FVII. This result merits the clinical translation.

### EXAMPLE 2

The in vivo properties of 18F-FVIIai for PET imaging was evaluated in a series of dogs with various spontaneous tumors using a clinical PET/CT scanner. The human 18F-FVIIai has high binding activity also to canine TF and demonstrated very useful in the visualization of TF positive tumors despite the presence of TF background expression and competition with endogenous FVII.

### References

1. van den Berg YW, Osanto S, Reitsma PH, Versteeg HH. The relationship between tissue factor and cancer progression: insights from bench and bedside. Blood. 2012; 119:924-32.
2. Ruf W, Yokota N, Schaffner F. Tissue factor in cancer progression and angiogenesis. Thromb Res. 2010;125 Suppl 2:S36-8.
3. Kasthuri RS, Taubman MB, Mackman N. Role of tissue factor in cancer. J Clin Oncol. 2009;27:4834-8.
4. Ruf W, Mueller BM. Thrombin generation and the pathogenesis of cancer. Semin Thromb Hemost. 2006;32 Suppl 1:61-8.
5. Ueno T, Toi M, Koike M, Nakamura S, Tominaga T. Tissue factor expression in breast cancer tissues: its correlation with prognosis and plasma concentration. Br J Cancer. 2000;83:164-70.
6. Seto S, Onodera H, Kaido T, et al. Tissue factor expression in human colorectal carcinoma: correlation with hepatic metastasis and impact on prognosis. Cancer. 2000;88:295-301.
7. Khorana AA, Ahrendt SA, Ryan CK, et al. Tissue factor expression, angiogenesis, and thrombosis in pancreatic cancer. Clin Cancer Res. 2007;13:2870-5.
8. Forster Y, Meye A, Albrecht S, Schwenzer B. Tissue factor and tumor: clinical and laboratory aspects. Clin Chim Acta. 2006;364:12-21.
9. Nitori N, Ino Y, Nakanishi Y, et al. Prognostic significance of tissue factor in pancreatic ductal adenocarcinoma. Clin Cancer Res. 2005;11:2531-9.
10. Kakkar AK, Lemoine NR, Scully MF, Tebbutt S, Williamson RC. Tissue factor expression correlates with histological grade in human pancreatic cancer. Br J Surg. 1995;82:1101-4.
11. Yu JL, May L, Lhotak V, et al. Oncogenic events regulate tissue factor expression in colorectal cancer cells: implications for tumor progression and angiogenesis. Blood. 2005; 105:1734-41.
12. Hu Z, Sun Y, Garen A. Targeting tumor vasculature endothelial cells and tumor cells for immunotherapy of human melanoma in a mouse xenograft model. Proc Natl Acad Sci USA. 1999;96:8161- 6.
13. Ngo CV, Picha K, McCabe F, et al. CNTO 859, a humanized antitissue factor monoclonal antibody, is a potent inhibitor of breast cancer metastasis and tumor growth in xenograft models. Int J Cancer. 2007;120:1261-7.
14. Versteeg HH, Schaffner F, Kerver M, et al. Inhibition of tissue factor signaling suppresses tumor growth. Blood. 2008;111:190-9.
15. Breij ECW, de Goeij BECG, Verploegen S, et al. An antibody-drug conjugate that targets tissue factor exhibits potent therapeutic activity against a broad range of solid tumors. Cancer Res. 2014;74:1214-26.
16. Sorensen BB, Persson E, Freskgård PO, et al. Incorporation of an active site inhibitor in factor VIIa alters the affinity for tissue factor. J Biol Chem. 1997;272:11863-8.
17. Tang G, Tang X, Wang X. A facile automated synthesis of N-succinimidyl 4-[18F]fluorobenzoate ([18F]SFB) for 18F-labeled cell-penetrating peptide as PET tracer. J Label Compd Radiopharm. 2010;53:543-7.
18. Erlandsson M, Nielsen CH, Jeppesen TE, et al. Synthesis and characterization of (18)F-labeled active site inhibited factor VII (ASIS). J Label Compd Radiopharm. 2015;58: 196-201.
19. Madsen J, Kristensen JB, Olsen OH, et al. Recombinant coagulation factor VIIa labelled with the fac-99 mTc(CO)3-core: synthesis and in vitro evaluation of a putative new radiopharmaceutical for imaging in acute bleeding lesion. J Label Compd Radiopharm. 2011;54:214-9.
20. Persson E. Influence of the gamma-carboxyglutamic acid-rich domain and hydrophobic stack of factor VIIa on tissue factor binding. Haemostasis. 1996;26 Suppl 1:31-4.
21. Nalla A, Buch I, Sigvardt M, Bodholdt RP, Kjaer A, Hesse B. (111)lndium labelling of recombinant activated coagulation factor VII: In vitro and preliminary in vivo studies in healthy rats. Int J Mol Imaging. 2012;2012:464810.
22. Temma T, Ogawa Y, Kuge Y, et al. Tissue factor detection for selectively discriminating unstable plaques in an atherosclerotic rabbit model. J Nucl Med. 2010;51:1979-86.
23. Hong H, Zhang Y, Nayak TR, et al. Immuno-PET of tissue factor in pancreatic cancer. J Nucl Med. 2012;53:1748-54.
24. Shi S, Hong H, Orbay H, et al. ImmunoPET of tissue factor expression in triple-negative breast cancer with a radiolabeled antibody Fab fragment. Eur J Nucl Med Mol Imaging. 2015;42:1295-303.
25. Viola-Villegas NT, Sevak KK, Carlin SD, et al. Noninvasive imaging of PSMA in prostate tumors with (89)Zr-labeled huJ591 engineered antibody fragments: the faster alternatives. Mol Pharmaceutics. 2014;11:3965-73.
26. Cirillo P, Golino P, Ragni M, et al. Long-lasting antithrombotic effects of a single dose of human recombinant, active site blocked factor VII: insights into possible mechanism(s) of action. J Thromb Haemost. 2003;1:992-8.
27. Erhardtsen E, Nilsson P, Johannessen M, Thomsen MS. Pharmacokinetics and safety of FFR-rFVIIa after single doses in healthy subjects. J Clin Pharmacol. 2001;41:880-5.
28. Petersen LC, Nørby PL, Branner S, et al. Characterization of recombinant murine factor VIIa and recombinant murine tissue factor: a human-murine species compatibility study. Thromb Res. 2005;116:75-85.
29. Müller M, Flössel C, Haase M, et al. Cellular localization of tissue factor in human breast cancer cell lines. Virchows Arch, B, Cell Pathol. 1993;64:265-9.
30. Saito Y, Hashimoto Y, Kuroda J-I, et al. The inhibition of pancreatic cancer invasion-metastasis cascade in both cellular signal and blood coagulation cascade of tissue factor by its neutralisation antibody. Eur J Cancer. 2011;47:2230-9.
31. Niu G, Li Z, Xie J, Le Q-T, Chen X. PET of EGFR antibody distribution in head and neck squamous cell carcinoma models. J Nucl Med. 2009;50:1116-23.
32. Beeby TL, Chasseaud LF, Taylor T, Thomsen MK. Distribution of the recombinant coagulation factor 125I-rFVIIa in rats. Thromb Haemost. 1993;70:465-8.
33. Knudsen T, Olsen OH, Petersen LC. Tissue factor and factor VIIa cross-species compatibility. Front Biosci (Landmark Ed). 2011;16:3196-215.
34. Jilma B, Marsik C, Mayr F, et al. Pharmacodynamics of active siteinhibited factor VIIa in endotoxin-induced coagulation in humans. Clinical Pharmacology & Therapeutics. 2002;72:403-10.
35. Vincent J-L, Artigas A, Petersen LC, Meyer C. A multicenter, randomized, double-blind, placebo-controlled, dose-escalation trial assessing safety and efficacy of active site inactivated recombinant factor VIIa in subjects with acute lung injury or acute respiratory distress syndrome. Crit Care Med. 2009;37:1874-80.
36. Neumaier B, Mottaghy FM, Buck AK, et al. Short Communication: (18)F-immuno-PET: Determination of anti-CD66 biodistribution in a patient with high-risk leukemia. Cancer Biother Radiopharm. 2008;23:819-24.

### SEQUENCE LISTING

<110> RIGSHOSPITALET
<120> Quantitative PET Imaging of Tissue Factor Expression Using 18F-labled Active Site Inhibited Factor VII
<130> P1422PC00
<150> PA 2016 70019
   <151> 2016-01-15
<160> 1
<170> PatentIn version 3.5
<210> 1
   <211> 406
   <212> PRT
   <213> Homo sapiens
<400> 1

## Claims

1. A positron-emitting 18-F labelled factor VIIa (FVIIa) imaging agent for use in a diagnosis by PET imaging of a Tissue Factor expressing tumor in a human, said agent comprising native human FVIIa or a variant thereof radiolabeled with 18-F, wherein the agent is to be administered in a dose of 50-400 MBq followed by PET scanning 1-6 hours after the agent has been administered, quantification through SUVmax and/or SUVmean for thereby obtaining a PET image for the diagnosis of tumor tissue factor status for use of diagnosis, treatment monitoring or as a companion diagnostics based on target-to-background ratio or absolute uptake (SUV).

2. The 18-F labelled factor VIIa (FVIIa) imaging agent for use according to claim 1, wherein the FVIIa is SEQ ID NO: 1 and active site inhibited factor VIIa (FVIIai) and modified in such a way that it is catalytically inactive.

3. The 18-F labelled factor VIIa (FVIIa) imaging agent for use according to claim 2, wherein FVIIai has been labelled with N-succinimidyl 4-¹⁸Ffluorobenzoate (18F-SFB) to produce:

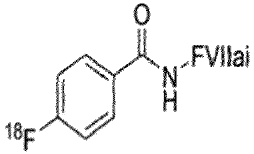

4. The 18-F labelled factor VIIa (FVIIa) imaging agent for use according to any one of the claims 1-3 to diagnose, stage or therapy monitoring in breast, gastric, esophageal, liver, colorectal and pancreatic cancer.

## Patentansprüche

1. Positronenabgebendes 18-F-markiertes Faktor Vlla (FVIIa)-Kontrastmittel zur Verwendung in einer Diagnostik durch PET-Bildgebung eines Gewebefaktorexpressionsgebenden Tumors in einem Mensch, wobei das Mittel natives humanes FVIIa oder eine mit 18-F radioaktiv markierte Variante davon umfasst, wobei das Mittel in einer Dosis von 50-400 MBq zu verabreichen ist, gefolgt von einem PET-Scanning 1-6 Stunden nach der Verabreichung des Mittels, Quantifizierung durch SUV-Höchstwert und/oder SUV-Mittelwert um dadurch ein PET-Bild zu Erlangen für die Diagnostik des Tumorgewebefaktorzustands zur Verwendung für Diagnostik, Therapieüberwachung oder als begleitende Diagnostika aufgrund eines Ziel-Hintergrund-Verhältnisses oder absoluter Aufnahmewerte (SUV).

2. 18-F-markiertes Faktor Vlla (FVIIa)-Kontrastmittel zur Verwendung nach Anspruch 1, wobei das FVIIa SEQ ID NO: 1 und an der aktiven Stelle gehemmter Faktor Vlla (FVIIai) ist, und so modifiziert ist, dass es katalytisch inaktiv ist.

3. 18-F-markiertes Faktor Vlla (FVIIa)-Kontrastmittel zur Verwendung nach Anspruch 2, wobei FVIIai mit N-Succinimidyl 4-¹⁸F-Fluorbenzoat (18F-SFB) markiert wurde, um 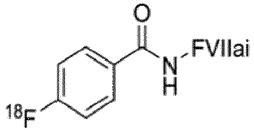 zu erzeugen.

4. 18-F-markiertes Faktor Vlla (FVIIa)-Kontrastmittel zur Verwendung nach einem jeglichen der Ansprüche 1-3 zur Diagnostizierung, Stadium- oder Therapieüberwachung in Brust-, Magen-, Speiseröhren-, Leber-, Kolorektal- und Bauchspeicheldrüsenkrebs.

## Revendications

1. Agent d'imagerie de facteur VIIa (FVIIa) émetteurs de positrons marqué au 18-F pour utilisation dans un diagnostic d'imagerie par TEP d'une tumeur d'expression de Facteur Tissulaire chez un humain, ledit agent comprenant du FVIIa humain natif ou une variante de celui-ci radiomarquée au 18-F, où l'agent doit être administré d'une dose de 50 à 400 MBq suivi d'une scintigraphie en TEP 1 à 6 heures après l'administration de l'agent, quantification par SUVmax et/ou SUVmean, obtenant ainsi une image TEP pour le diagnostic de l'état du facteur tissulaire tumoral pour l'utilisation de diagnostic, la supervision du traitement ou en tant que des diagnostics compagnons basés sur un ratio cible-arrière-plan ou absorption absolue (SUV).

2. Agent d'imagerie de facteur VIIa (FVIIa) marqué au 18-F pour utilisation selon la revendication 1, où le FVIIa est SEQ ID NO: 1 et un facteur VIIa (FVIIai) à site actif inhibé et modifié d'une telle manière qu'il est catalytiquement inactif.

3. Agent d'imagerie de facteur VIIa (FVIIa) marqué au 18-F pour utilisation selon la revendication 2, où le FVIIai a été marqué avec du N-succinimidyl 4-¹⁸Ffluorobenzoïque (18F-SFB) pour produire:

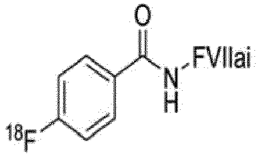

4. Agent d'imagerie de facteur VIIa (FVIIa) marqué au 18-F pour utilisation selon l'une quelconque des revendications 1 à 3 pour le diagnostic, la surveillance d'étape où la surveillance du traitement du cancer du sein, du cancer gastrique, du cancer oesophagien, du cancer du foie, du cancer colorectal et du cancer pancréatique.
